(19) Europäisches Patentamt
European Patent Office
Office européen des brevets

(11) Veröffentlichungsnummer: **0 011 178**
**B1**

(12) **EUROPÄISCHE PATENTSCHRIFT**

(45) Veröffentlichungstag der Patentschrift: **24.03.82**

(51) Int. Cl.³: **C 07 H 17/04,** C 12 P 19/60, A 61 K 31/72 // C12R1/045

(21) Anmeldenummer: **79104202.1**

(22) Anmeldetag: **29.10.79**

(54) **Antibiotikum, seine Herstellung sowie seine Verwendung als Arzneimittel.**

(30) Priorität: **10.11.78 DE 2848793**

(43) Veröffentlichungstag der Anmeldung: **28.05.80 Patentblatt 80/11**

(45) Bekanntmachung des Hinweises auf die Patenterteilung: **24.03.82 Patentblatt 82/12**

(84) Benannte Vertragsstaaten: **AT BE CH DE FR GB IT NL SE**

(56) Entgegenhaltungen:
**US-A-4031 207**
**US-A-4031 208**

(73) Patentinhaber: **BAYER AG, Zentralbereich Patente, Marken und Lizenzen, D-5090 Leverkusen 1, Bayerwerk (DE)**

(72) Erfinder: **Bauer, Klaus, Dr., Am Dorpweiher 66, D-5600 Wuppertal 1 (DE)**
Erfinder: **Gau, Wolfgang, Dr., Am Eckbusch 39/56, D-5600 Wuppertal 1 (DE)**
Erfinder: **Kaufmann, Wilfried, Dr., Zur Waldesruh 35, D-5600 Wuppertal 11 (DE)**
Erfinder: **Pfitzner, Jörg, Dr., Claudiusweg 3, D-5600 Wuppertal 1 (DE)**
Erfinder: **Scheer, Martin, Dr., Claudiusweg 9, D-5600 Wuppertal 1 (DE)**
Erfinder: **Schröder, Theo, Dr., Am Eckbusch 39/82, D-5600 Wuppertal 1 (DE)**

**EP 0 011 178 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Antibiotikum, seine Herstellung sowie seine Verwendung als Arzneimittel

Die vorliegende Erfindung betrifft ein neues Antibiotikum, ein mikrobiologisches Verfahren zu seiner Herstellung aus einem Actinoplanaceenstamm sowie seine Verwendung als Arzneimittel, insbesondere als antimikrobielles Mittel in der Human- und Tiermedizin sowie als Mittel zur Förderung des Wachstums und der Steigerung der Futterverwertung bei Tieren.

Es ist bereits bekannt geworden, dass eine Reihe von Antibiotika mikrobieller Herkunft antimikrobielle Wirkungen besitzen. Diese Antibiotika sind teilweise in ihren Wirkungsspektren nicht voll befriedigend. Sie weisen häufig noch weitere Nachteile auf. β-Lactamantibiotika werden oft durch Penicillinase inaktiviert. Chloramphenicol, Tetracycline und Streptomicin zeigen in vielen Fällen erhebliche unerwünschte Nebenwirkungen (vgl. Walter Heilmeyer, „Antibiotika-Fibel", Georg Thieme Verlag, Stuttgart, 3. Auflage, 1969, S. 248, 278-280 und 311-319).

Es wurde nun gefunden, dass man ein neues Antibiotikum erhält, wenn man den Actinoplanesstamm SE-73/34 d (Kl.) in einem Nährmedium züchtet und das Antibiotikum nch bekannten Methoden aus dem Nährmedium isoliert.

Bei dem obengenannten Actinoplanesstamm handelt es sich um eine Spontanmutante des Actinoplanesstammes SE-73 (ATCC 31058).

Weiterhin wurde gefunden, dass das neue Antibiotikum eine starke antimikrobielle Wirkung aufweist und ausserdem die Eigenschaft besitzt, bei Tieren das Wachstum und die Futterverwertung zu verbessern.

Aus der US-A Nr. 4031208 ist ein Antibiotikum bekannt, das sich vom erfindungsgemässen besonders hinsichtlich seines UV-Spektrums, aber auch in anderen Parametern, z.B. Schmelzpunkt und Sauerstoffgehalt, unterscheidet.

Die erfindungsgemässe Verbindung wird durch die folgenden chemischen und physikalischen Eigenschaften charakterisiert:

1) Elementaranalyse und Summenformel:

Berechnet für C 73 H 110 O 37 (1578,6)

Berechnet: C 55,5 H 7,0 O 37,5 $4OCH_3 = 7,9$

Gefunden*: C 55,6 H 6,9 O 37,8** $OCH_3 = 7,9$

* Nach 4 Tagen Trocknen im Hochvakuum bei 100°C.

** Aus der Differenz berechnet.

Es muss darauf hingewiesen werden, dass bei so grossen Molekülen die Fehlerbreite der Elementaranalyse es nicht erlaubt, eine genaue Summenformel zu berechnen (R. P. Woodward, „Angew. Chem.", *69*, 50-51, 1957). Die gesamte Summenformel kann daher einen gewissen Fehler aufweisen.

2) Schmelzpunkt: 164-172°C (unter Zersetzung).

3) Ultraviolettabsorptionsspektrum:

Das UV-Spektrum des Antibiotikums wird in Fig. 1 wiedergegeben (Abszisse: Wellenlänge in mμ und Ordinate: Extinktion):

$\lambda_{max.}$ = 365 nm (C = 0,64 mg in 50 ml Methanol)

$$\left[E \frac{1\%}{cm}\right]_{365\ nm} = 406$$

4) IR-Absorptionsspektrum:

Das IR-Absorptionsspektrum des Antibiotikums wird in Fig. 2 wiedergegeben (Abszisse: Wellenzahl in $cm^{-1}$ Ordinate: Absorption). Es zeigt, wenn es zu KBr-Presslingen verpresst wird, bei folgenden Wellenlängen (ausgedrückt in reziproken Zentimetern) Absorptionsbanden:

| Wellenlänge | Intensität | Wellenlänge | Intensität |
|---|---|---|---|
| 3450 | s | 1238 | s |
| 2980 | m | 1200 | s |
| 2940 | s | 1120 | s |
| 2900 | m | 1025 | s |
| 1700 | s | 945 | s |
| 1640 | s | 905 | s |
| 1598 | m | 870 | m |
| 1580 | m | 840 | m |
| 1450 | s | 820 | m |
| 1370 | s | 785 | m |
| 1300 | s | | |

s = stark, m = mittel.

5) $^1$H-Kernresonanzspektrum, angegeben in Teilen pro Million (ppm) und Schwingungen pro Sekunde (Hz) gemäss Fig. 3.

6) $^{13}$C-Kernresonanzspektrum gemäss Fig. 4.

Das $^{13}$C-Kernresonanzspektrum zeigt die folgenden Signale, angegeben in Teilen pro Million (ppm) und Schwingungen pro Sekunde (Hz), in ihren relativen Intensitäten an:

| Signal Nr. | Intensität | Signallage Hz | Signallage ppm |
|---|---|---|---|
| 1 | 60 | 4214,3 | 167,243 |
| 2 | 28 | 3673,3 | 145,773 |
| 3 | 26 | 3566,9 | 141,550 |
| 4 | 40 | 3473,5 | 137,843 |
| 5 | 81 | 3442,5 | 136,612 |
| 6 | 35 | 3409,2 | 135,293 |
| 7 | 31 | 3320,6 | 131,777 |
| 8 | 28 | 3262,6 | 129,472 |
| 9 | 141 | 3238,3 | 128,511 |
| 10 | 62 | 3231,2 | 128,229 |
| 11 | 51 | 3225,3 | 127,994 |
| 12 | 129 | 3187,2 | 126,481 |
| 13 | 56 | 3052,3 | 121,129 |
| 14 | 65 | 3034,2 | 120,410 |
| 15 | 96 | 3013,0 | 119,568 |
| 16 | 30 | 3002,5 | 119,151 |
| 17 | 30 | 2630,2 | 104,379 |
| 18 | 26 | 2560,4 | 101,606 |
| 19 | 28 | 2515,9 | 99,842 |
| 20 | 26 | 2435,2 | 96,640 |
| 21 | 30 | 2418,1 | 95,961 |
| 22 | 31 | 2397,8 | 95,154 |
| 23 | 26 | 2173,3 | 85,247 |

| Signal Nr. | Intensität | Signallage Hz | Signallage ppm |
|---|---|---|---|
| 24 | 45 | 2074,4 | 82,322 |
| 25 | 36 | 2062,0 | 81,329 |
| 26 | 33 | 2049,2 | 81,321 |
| 27 | 31 | 2039,8 | 80,947 |
| 28 | 36 | 2031,4 | 80,614 |
| 29 | 46 | 2004,3 | 79,561 |
| 30 | 678 | 1971,1 | 78,222 |
| 31 | 63 | 1955,6 | 77,606 |
| 32 | 836 | 1939,3 | 76,958 |
| 33 | 27 | 1930,6 | 76,617 |
| 34 | 25 | 1927,2 | 76,482 |
| 35 | 29 | 1923,1 | 76,319 |
| 36 | 753 | 1907,6 | 75,703 |
| 37 | 55 | 1898,7 | 75,349 |
| 38 | 91 | 1895,6 | 75,226 |
| 39 | 55 | 1877,9 | 74,523 |
| 40 | 46 | 1848,0 | 73,335 |
| 41 | 47 | 1843,5 | 73,160 |
| 42 | 53 | 1828,1 | 72,549 |
| 43 | 80 | 1812,9 | 71,945 |
| 44 | 37 | 1806,1 | 71,674 |
| 45 | 56 | 1791,1 | 71,079 |
| 46 | 55 | 1777,1 | 70,522 |
| 47 | 46 | 1769,2 | 70,209 |
| 48 | 41 | 1763,9 | 69,998 |
| 49 | 48 | 1745,9 | 69,287 |
| 50 | 38 | 1730,3 | 68,667 |
| 51 | 25 | 1714,1 | 68,023 |
| 52 | 55 | 1564,1 | 62,072 |
| 53 | 43 | 1559,0 | 61,862 |
| 54 | 75 | 1490,2 | 59,140 |
| 55 | 28 | 509,7 | 20,229 |
| 56 | 55 | 457,1 | 18,140 |
| 57 | 67 | 452,7 | 17,965 |
| 58 | 53 | 407,5 | 16,173 |
| 59 | 183 | 0,5 | 0,019 |

Darüberhinaus sind in Fig. 4 noch 3 Signale mit geringer Intensität sichtbar, die vom Datensystem nicht erfasst werden. Sie liegen bei 35,74, 38,36 und 40,50 ppm.

7) Das Antibiotikum ist gut in Chloroform, Aceton, Essigsäureäthylester, Acetonitril, Dimethylformamid, Dimethylsulfoxid und niederen Alkoholen, z.B. Methanol, und Äthanol gut löslich; dagegen ist es in Wasser, Diäthyläther, Petroläther und Cyclohexan weniger löslich.

8) Das erfindungsgemässe Antibiotikum ist ein amorpher, blassgelber Neutralstoff, der bei der Elektrophorese nicht wandert. Bei kräftiger saurer Hydrolyse werden verschiedene reduzierende Zucker abgespalten (z.B. mit 10 Gew.%iger wässriger Schwefelsäure bei 80°C). Dadurch ist es leicht möglich, das Antibiotikum bei der Dünnschichtchromatographie auf einer Kieselgelplatte mit Zuckerreagenzien (z.B. mit Anisaldehydschwefelsäure, Thymolschwefelsäure, Vanillinperchlorsäure oder Dimedonphosphorsäure) oder mit Molybdatophosphorsäure durch charakteristische Färbungen sichtbar zu machen. Einige Färbungen, die zur Identifikation dienen können, sind in Tabelle 1 aufgeführt.

Tabelle 1

| Nr. | Reagenz* | Farbe | Untergrund |
|---|---|---|---|
| 1 | 10% $H_2SO_4$ | Braun | Weiss |
| 2 | Jod | Braun | Braungelb |
| 3 | Molybdatophosphorsäure | Blaugrün | Blassgrün |
| 4 | Silbernitrat-$NH_3$ | Braun | Hellbraun |
| 5 | Thymol-$H_2SO_4$ | Rotviolett | Farblos |
| 6 | Dimedon-$H_3PO_4$ | Grün (UV-Licht-gelb) | Farblos |
| 7 | Anisaldehyd-$H_2SO_4$ | Grün | Farblos |
| 8 | Vanillin-$HClO_4$ | Braun | Farblos |

* Die Reagentien wurden nach den üblichen Vorschriften (vgl. E. Stahl, „Dünnschichtchromatographie", 2. Auflage, Springer Verlag, Berlin, Heidelberg, New York, 1967) angesetzt.

9) Die $R_F$-Werte des erfindungsgemässen Antibiotikums auf neutralen Kieselgelplatten (Fa. Merck, Darmstadt, BRD) im Vergleich zu Erythromycinbase in verschiedenen Laufmitteln gibt Tabelle 2 wieder.

Tabelle 2

| Nr. | Laufmittel (Volumenteile) | $R_F$-Wert neues Antibiotikum | $R_F$-Wert Erythromycinbase |
|---|---|---|---|
| | Chloroform + Methanol | | |
| 1 | 95 + 5 | 0,00 | 0,00 |
| 2 | 90 + 10 | 0,27 | 0,08 |
| 3 | 80 + 20 | 0,59 | 0,21 |
| 4 | 50 + 50 | 0,85 | 0,26 |
| 5 | Chloroform + Methanol + Ammoniak* | | |
| | 40 + 6 + 1 | 0,34 | 0,62 |
| 6 | Methanol | 0,83 | 0,25 |
| 7 | Butanol + Eisessig + Wasser | | |
| | 60 + 20 + 20 | 0,55 | 0,39 |

* 25 Gew.% Ammoniak enthaltende wässrige Lösung.

Das in Fig. 3 gezeigte 220 MHz-$^1$H-Kernresonanzspektrum wurde an einer Lösung des Antibiotikums in deuteriertem Pyridin mit Tetramethylsilan als innerem Standart auf einem HR-SC-Spektrometer der Fa. Varian Associates, Paolo Alto, Californien, USA, aufgenommen.

Das $^{13}$C-Kernresonanzspektrum (Fig. 4) wurde an einer Lösung des Antibiotikums in deuteriertem Chloroform auf einem XL-100-Spektrophotometer (15") der Fa. Varian bei 25,2 MHz unter Protonenrauschentkopplung gemessen.

Die IR-Bandenintensitäten werden mit β, m und w bezeichnet. Eine s-Bande weist mindestens 2/3 der Intensität der stärksten Bande im Spektrum, eine m-Bande eine Intensität im Bereich zwischen 1/3 und 2/3 der stärksten Bande und eine w-Bande weniger als 1/3 der Intensität der stärksten Bande auf. Diese Schätzungen sind auf der Basis der prozentualen Durchlässigkeit gemacht.

Die vorliegenden physikalisch-chemischen und spektroskopischen Daten des Antibiotikums stehen mit der folgenden Strukturformel im Einklang:

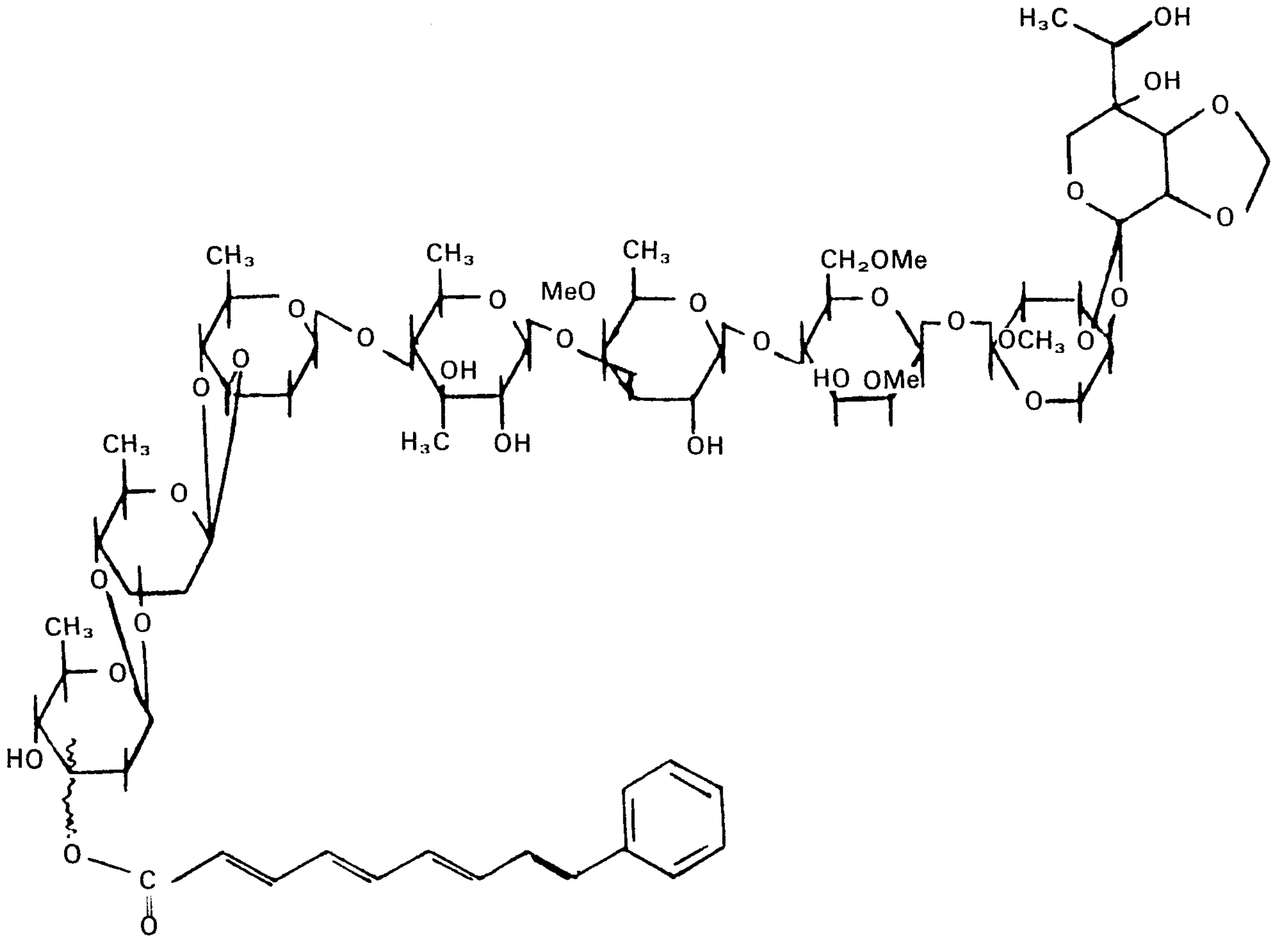

Überraschenderweise wird das erfindungsgemässe Antibiotikum durch die obengenannte Spontanmutante 34d (Kl.) des Actinoplanesstammes SE-73 gebildet und kann aus dem Kulturmedium in guter Ausbeute isoliert werden. Weiterhin ist es überraschend, dass das erfindungsgemässe Antibiotikum eine starke antimikrobielle, insbesondere antibakterielle Wirkung auch gegenüber gramnegativen Erregern aufweist, ohne die oben dargelegten Nachteile bekannter Antibiotika zu zeigen. Das neue Antibiotikum weist auch überraschenderweise die Eigenschaft auf, bei Tieren das Wachstum zu fördern und die Futterverwertung zu verbessern. Die vorliegende Erfindung stellt daher eine Bereicherung der Pharmazie und der Technik dar. Der erfindungsgemässe Stamm Actinoplanes SE-73/34d (Kl.) gehört der Klasse der Schizomyceten, der Ordnung der *Actinomycetales*, der Familie der *Actinoplanaceae* und der Gattung der Actinoplanes an. Er wurde unter der Nummer ATCC 31440 bei American Type Culture Collection, 12301 Parklawn Drive, Rockville, Maryland 20852, USA, am 4. Oktober 1978 hinterlegt. Der Stamm hat die folgenden Merkmale:

Die Kulturmerkmale auf verschiedenen Nährböden (nach 14d bei einer Wachstumstemperatur von 20 bis 30°C beobachtet) gehen aus der nachstehenden Aufstellung hervor.

| | | |
|---|---|---|
| Czapek-Agar | W | ++ |
| | SM | orange |
| | LP | schwach bräunlich-gelb |
| | Spg. | — |
| CPC | W | ++ |
| (Casamino- | SM | orange bis orange-braun |
| pepton/ | LP | braun |
| Czapek-Agar) | Spg. | — |
| Milchagar | W | ++ |
| | SM | orange |

| | | |
|---|---|---|
| Milchagar | LP | goldbraun |
| | Spg. | — |
| | Casein peptonisiert | |
| Tyrosinagar | W | + |
| | SM | braun |
| | LP | braun |
| | Spg. | — |
| | Tyrosinkristalle nicht aufgelöst | |
| Melaninbildung | | + |
| Milchpeptonisierung | | + |

W = Wachstum
SM = Substratmycel
LP = Lösliches Pigment
Spg. = Bildung von Sporangien
— = Fehlen
\+ = ausgeprägtes Vorhandensein
++ = sehr ausgeprägtes Vorhandensein

Das erfindungsgemässe Verfahren kann mit Hilfe fester, halbfester, oder flüssiger Nährmedien durchgeführt werden. Bevorzugt werden wässrigflüssige Nährmedien verwendet.

Die Beimpfung der Nährmedien erfolgt nach allgemein üblichen Methoden, z.B. über Schrägröhrchen oder Kolbenkulturen. Die Kultur erfolgt unter aeroben Bedingungen und kann gemäss den allgemein üblichen Methoden, beispielsweise unter Verwendung von Schüttelkulturen in Schüttelkolben, von luftbewegten Kulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Kultur kontinuierlich oder diskontinuierlich durchzuführen. Vorzugsweise wird diskontinuierlich gearbeitet.

Die Kultur kann in allen Nährmedien durchgeführt werden, welche bekannterweise zur Kultivierung von Mikroorganismen der Ordnung der *Actinomycetales* verwendet werden. Das Nährmedium muss eine oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als Kohlenstoffquellen kommen alle üblichen Kohlenstoffquellen infrage. Beispielsweise seien Stärke, Melasse, Molkepulver, Dextrin, Zucker, wie Saccharose, Maltrose, Glucose, Lactose, Sorbit und Glycerin, genannt. Als Stickstoffquellen kommen alle üblichen organischen und anorganischen Stickstoffquellen infrage. Beispielsweise seien Sojabohnenmehl, Baumwollsamenmehl, Linsenmehl, Erbsenmehl, lösliche und unlösliche pflanzliche Proteine, Maisquellwasser, Hefeextrakte, Peptone und Fleischextrakt sowie Ammoniumsalze und Nitrate, z.B. $NH_4Cl$, $(NH)_2SO_4$, $NaNO_3$ und $KNO_3$ aufgeführt. Die Mineralsalze, welche im Nährmedium enthalten sein sollten, liefern z.B. folgende Ionen:

$Mg^{++}$, $Na^+$, $K^+$, $Ca^{++}$, $NH_4^+$, $Cl^-$, $SO_4^{--}$, $PO_4^{---}$ und $NO_3^-$

sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthält, ist es zweckmässig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen.

Der pH-Wert der wachsenden Kulturen sollte vorzugsweise zwischen etwa 6 und etwa 8, insbesondere zwischen 6,5 und 7,5 gehalten werden. Ein zu starker pH-Abfall in den sauren Bereich kann durch Zusätze einer organischen oder anorganischen Base, vorzugsweise von $CaCO_3$, vermieden werden. Wie in der Fermentationstechnologie üblich, kann auch eine automatische pH-Regulierung durchgeführt werden, bei der sterile organische oder anorganische Säure, z.B. $H_2SO_4$, oder sterile Lauge, z.B. NaOH, in Abständen in die Kulturlösung eingespritzt werden.

Es ist zweckmässig, sicherzustellen, dass die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 20 und etwa 40°C, vorzugsweise zwischen 25 und 35°C liegen, besonders bevorzugt liegt sie bei etwa 28°C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, dass die Menge des sich in der Kulturbrühe anreichernden Antibiotikums im allgemeinen ihr Maximum etwa 2 bis 12, vorzugsweise 5 bis 8d nach Züchtungsbeginn erreicht.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefässe sowie der für die Belüftung notwendigen Luft. Zur Sterilisation der Vorrichtungen können z.B. die Dampf- als auch die Trockensterilisation verwendet werden.

Falls bei der Kultivierung in unerwünschter Menge Schaum entsteht, können die üblichen chemischen Schaumdämpfungsmittel, z.B. flüssige Fette und Öle, Öl/Wasser-Emulsionen, Paraffine, höhere Alkohole, wie Octodecanol, Siliconöle, Polyoxyäthylen- bzw. Polyoxypropylenverbindungen zugesetzt werden. Schaum kann auch mit Hilfe der üblichen mechanischen Vorrichtungen (welche z.B. Zentrifugalkräfte benutzen) gedämpft oder beseitigt werden.

Das erfindungsgemässe Antibiotikum kann aus dem Mycel und/oder aus dem Kulturmedium nach allgemein üblichen physikalisch-chemischen Methoden isoliert werden. Die Isolierung kann z.B.

gemäss den üblichen Extraktionsverfahren, Fällungsverfahren und/oder Chromatographieverfahren erfolgen. Das isolierte Antibiotikum kann mit Hilfe der genannten Methoden auch feingereinigt werden. Für viele Fälle ist jedoch eine Feinreinigung nicht erforderlich, da die gegebenenfalls vorhandenen Verunreinigungen die Wirksamkeit des Antibiotikums nicht nachteilig beeinflussen. Bei allen Isolierungs- und Reinigungsoperationen ist darauf zu achten, dass pH-Werte von 7,0 oder mehr, vorzugsweise zwischen 7,0 bis 9,0, eingehalten werden. Zur Erhöhung des pH-Wertes können anorganische und organische Basen verwendet werden, z.B. Ammoniak, Alkali- und Erdalkalihydroxide, Alkali- und Erdalkalicarbonate und Hydrogencarbonate, z.B. KOH, NaOH, $Na_2CO_3$, $CaCO_3$, Trialkylamine, wie Triäthylamin oder Morpholin und Pyridin. Um bei den obengenannten Isolierungs- und Reinigungsmethoden die Fraktionen herauszufinden, in welchen das erfindungsgemässe Antibiotikum in höchster Konzentration bzw. Reinheit vorliegt, können die üblichen physikalischen-chemischen Methoden, z.B. Messen der UV-Bande bei 365 nm, der $R_F$-Werte oder vorzugsweise die Untersuchung der antimikrobiellen Aktivität herangezogen werden. Besonders günstig ist im vorliegenden Fall die Untersuchung der Aktivität gegen *Bacillus subtilis*, beispielsweise *Bacillus subtilis* ATCC 6633 mit Hilfe des üblichen Plattentests (vgl. z.B. P. Klein, „Bakteriologische Grundlagen der chemotherapeutischen Laboratoriumspraxis", Springer Verlag, Göttingen, 1957, S. 86 ff.).

Die Isolierung und Reinigung des erfindungsgemässen Antibiotikums kann z.B. im Falle, dass ein flüssiges, wässriges Nährmedium verwendet wird, wie folgt vorgenommen werden:

Zur Kulturbrühe einschliesslich Mycelium wird ein mit Wasser mischbares organisches Lösungsmittel gegeben und gut vermischt, wodurch einerseits Wirkstoff aus dem Mycelium extrahiert und andererseits eine Klärung der Kulturbrühe erreicht wird.

Als Lösungsmittel können z.B. niedere Alkanole wie Methanol, Äthanol, n- und i-Propanol oder t-Butanol, Dimethylformamid Tetrahydrofuran und besonders bevorzugt Aceton, verwendet werden. Die Menge des Lösungsmittels kann in weiten Grenzen variiert werden. Bevorzugt wird etwa das gleiche Volumen, wie es die Kulturbrühe hat, zugesetzt. Anschliessend werden die nicht gelösten Bestandteile (Mycelium, ausgefällte Proteine usw.) durch Filtration, Zentrifugation, Abdekantieren usw. abgetrennt.

Die wässrig-organische Lösung wird zweckmässigerweise im Vakuum etwa auf das Volumen des eingesetzten Kulturmediums eingeengt.

Gegebenenfalls wird mit einer Base (vgl. oben), vorzugsweise mit NaOH, ein pH-Wert von über 7,0, z.B. ein pH-Wert von 9,0, eingestellt. Die so erhaltene Lösung soll im folgenden als Lösung I bezeichnet werden.

Aus der Lösung I kann das erfindungsgemässe Antibiotikum mit Hilfe von üblichen Extraktionsverfahren, Fällungsverfahren und/oder Chromatographieverfahren isoliert und gegebenenfalls gereinigt werden. Die Chromatographie kann in Form der Säulenchromatographie oder der präparativen Schichtchromatographie durchgeführt werden. Als Adsorptionsmittel können alle üblichen (nicht sauren) anorganischen oder organischen Adsorptionsmittel eingesetzt werden, wie z.B. Aluminiumoxid, Kieselgel, Magnesiumsilikat Aktivkohle, Cellulose, Cellulosederivate, Kunstharze, wie Polyamide, Derivate von Polyamiden usw. z.B. acetyliertes Polyamid oder Dextrangele. Als Laufmittel bei der präparativen Dünnschichtchromatographie können die verschiedensten Lösungsmittel oder Lösungsmittelgemische verwendet werden, in welchen das erfindungsgemässe Antibiotikum löslich ist. Bevorzugt wird ein Gemisch aus Chloroform und Methanol (z.B. 9:1 Volumenteile) eingesetzt. Auch als Laufmittel für die Säulenchromatographie können Lösungsmittel bzw. Lösungsmittelgemische verwendet werden, in welchen das erfindungsgemässe Antibiotikum löslich ist. Beispielhaft seien Tetrachlorkohlenstoff, Methylenchlorid und bevorzugt Chloroform genannt.

Bevorzugt werden zur Isolierung des erfindungsgemässen Antibiotikums Extraktionsverfahren, gegebenenfalls in Kombination mit Chromatographieverfahren und Fällungsverfahren verwendet. Bei der Durchführung der Extraktionsschritte ist darauf zu achten, dass je nach dem, ob das erfindungsgemässe Antibiotikum in der wässrigen oder organischen Phase vorhanden sein soll, die Extraktionsmittel so gewählt werden, dass in ihnen das Antibiotikum schwer bzw. leicht löslich ist.

Das Extraktionsverfahren kann z.B. wie folgt durchgeführt werden:

Die Lösung I wird mit in Wasser nicht mischbaren organischen Lösungsmitteln nach üblichen Methoden (Schütteln, Gegenstromverfahren usw.) extrahiert. Als Lösungsmittel können die üblichen Extraktionsmittel, z.B. Ester, wie Äthylacetat und Butylacetat, höhere Alkohole wie Amylalkohole, z.B. n-Amylalkohol, in Wasser nicht mischbare Ketone, z.B. Methylisobutylketon, chlorierte niedere Kohlenwasserstoffe, z.B. Chloroform oder Methylenchlorid eingesetzt werden. Bevorzugt werden Äthylacetat und Butylacetat, insbesondere Äthylacetat verwendet.

Wenn bei der Extraktion der Lösung I Äthylacetat (und/oder Butylacetat) eingesetzt werden, wird die wässrige Phase verworfen, da sich das erfindungsgemässe Antibiotikum in der organischen Phase befindet.

Die organische Phase wird z.B. auf etwa 1/10-1/30 des ursprünglichen Volumens eingeengt. Anschliessend wird das erfindungsgemässe Antibiotikum durch die Zugabe eines organischen Fällungsmittels (Lösungsmittel), in welchem das erfindungsgemässe Antibiotikum schwer löslich ist, z.B. von Diäthyläther oder von gesättigten geradkettigen, verzweigten oder cyclischen Kohlenwasserstoffen, z.B. Petroläthern, n-Hexan, oder Cyclohexan nach den üblichen Methoden ausgefällt.

Das rohe Antibiotikum kann entweder durch die flüssig-flüssig-Verteilung (z.B. nach der Methode von Craig) oder gewünschtenfalls nach den üblichen chromatographischen Methoden (Säulenchromatographie, präparative Dünnschichtchromatographie) feingereinigt werden, wobei als Adsorptionsmittel, wie bereits erwähnt, die üblichen nicht sauren anorganischen und organischen Adsorptionsmittel verwendet werden können, z.B. Aluminiumoxid, Kieselgel, Magnesiumsilikat, Aktivkohle, Cellulose, Cellulosederivate, Kunstharz wie Polyamide, Derivate von Polyamiden, z.B. acetyliertes Polyamid, Dextrangele, Ionenaustauscher usw.

Wenn bei der Extraktion der Lösung I nicht Äthylacetat oder Butylacetat, sondern ein anderes Lösungsmittel, z.B. n-Butanol, verwendet wird, wird die wässrige Phase verworfen, da sich das erfindungsgemässe Antibiotikum überwiegend in der organischen Phase befindet. In diesem Falle wird das organische Lösungsmittel vorzugsweise auf etwa 1/10-1/30 des ursprünglichen Volumens eingeengt und durch die Zugabe eines geeigneten organischen Fällungsmittels, in welchem das erfindungsgemässe Antibiotikum schwer löslich ist, z.B. von Diäthyläther oder von gesättigten geradkettigen oder verzweigten oder cyclischen Kohlenwasserstoffen, z.B. Petroläther, n-Hexan oder Cyclohexan nach den üblichen Methoden gefällt. Der Niederschlag wird in Wasser aufgelöst und die Lösung gefriergetrocknet. Das erhaltene Rohprodukt kann, gegebenenfalls durch Extraktion mit Äthylacetat oder Butylacetat, worin unerwünschte Begleitstoffe zum grössten Teil nicht löslich sind, angereichert werden und nach den üblichen chromatographischen Methoden, z.B. durch Säulenchromatographie, präparative Dünnschichtchromatographie mit Hilfe der üblichen Adsorptionsmittel (vgl. auch die obigen Ausführungen) oder durch die flüssig-flüssig-Verteilung gereinigt werden. Zur Reinigung kann auch, wie bereits oben erwähnt, aus einer Lösung des Rohprodukts in einem organischen Lösungsmittel (z.B. Chloroform und Methylenchlorid) das erfindungsgemässe Antibiotikum mit Hilfe eines geeigneten organischen Fällungsmittels, in welchem das erfindungsgemässe Antibiotikum schwer löslich ist. z.B. von Diäthyläther, gesättigten geradkettigen oder verzweigten oder cyclischen Kohlenwasserstoffen, z.B. Petroläther, n-Hexan und Cyclohexan, ausgefällt werden.

Aus seinen Lösungen kann das Antibiotikum nach den üblichen Methoden, z.B. durch Verdampfen des Lösungsmittels, Gefriertrocknung usw. erhalten werden.

Zur vorliegenden Erfindung gehören pharmazeutische Zubereitungen, die neben nichttoxischen, inerten pharmazeutisch geeigneten Trägerstoffen den erfindungsgemässen Wirkstoff enthalten oder die aus dem erfindungsgemässen Wirkstoff bestehen, sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z.B. Tabletten, Dragées, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und formulierungshilfsmittel jeder Art zu verstehen.

Als bevorzugte pharmazeutische Zubereitungen seien Tabletten, Dragées, Kapseln, Pillen, Granulate, Suppositorien, Lösungen, Suspensionen und Emulsionen, Pasten, Salben, Gele, Cremes, Lotions, Puder und Sprays genannt.

Tabletten, Dragées, Kapseln, Pillen und Granulate können den oder die Wirkstoffe neben den üblichen Trägerstoffen enthalten, wie

a) Füll- und Streckmittel, z.B. Stärken, Milchzucker, Rohrzucker, Glucose, Mannit und Kieselsäure,

b) Bindemittel, z.B. Carboxymethylcellulose, Alginate, Gelatine, Polyvinylpyrrolidon,

c) Feuchthaltemittel, z.B. Glycerin,

d) Sprengmittel, z.B. Agar-Agar, Calciumcarbonat und Natriumcarbonat,

e) Lösungsverzögerer, z.B. Paraffin,

f) Resorptionsbeschleuniger, z.B. quaternäre Ammoniumverbindungen,

g) Netzmittel, z.B. Cetylalkohol, Glycerinmonostearat,

h) Adsorptionsmittel, z.B. Kaolin und Bentonit und

i) Gleitmittel, z.B. Talkum, Calcium- und Magnesiumstearat und feste Polyäthylenglykole oder Gemische der unter (a)-(i) aufgeführten Stoffe.

Die Tabletten, Dragées, Kapseln, Pillen und Granulate können mit den üblichen gegebenenfalls Opakisierungsmittel enthaltenden Überzügen und Hüllen versehen sein und auch so zusammengesetzt sein, dass sie den Wirkstoff nur oder bevorzugt in einem bestimmten Teil des Intestinaltraktes, gegebenfalls verzögert abgeben, wobei als Einbettungsmassen z.B. Polymersubstanzen und Wachse verwendet werden können.

Der Wirkstoff kann gegebenenfalls mit einem oder mehreren der oben angegebenen Trägerstoffe auch in mikroverkapselter Form vorliegen.

Suppositorien können neben dem Wirkstoff die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem Wirkstoff die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem Wirkstoff die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorofluorkohlenwasserstoffe enthalten.

Lösungen und Emulsionen können neben dem Wirkstoff die üblichen Trägerstoffe, wie Lösungsmittel, Lösungsvermittler und Emulgatoren, z.B. Wasser, Äthylalkohol, Isopropyalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem Wirkstoff die üblichen Trägerstoffe, wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin enthalten.

Die therapeutisch wirksame Verbindung soll in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können ausser dem erfindungsgemässen Wirkstoff auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des Wirkstoffs mit dem oder den Trägerstoffen.

Zur vorliegenden Erfindung gehört auch die Verwendung des erfindungsgemässen Wirkstoffes sowie von pharmazeutischen Zubereitungen, die den erfindungsgemässen Wirkstoff enthalten, in der Human- und Veterinärmedizin zur Verhütung, Besserung und/oder Heilung der oben angeführten Erkrankungen.

Der Wirkstoff oder die pharmazeutischen Zubereitungen können lokal, oral, parenteral, intraperitoneal und/oder rectal, vorzugsweise oral und parenteral, insbesondere intramuskulär oder intravenös, gegebenenfalls auch als Dauertropfinfusion, appliziert werden.

Im allgemeinen hat es sich sowohl in der Human- als auch in der Veterinärmedizin als vorteilhaft erwiesen, den erfindungsgemässen Wirkstoff bei oraler oder parenteraler Applikation in Gesamtmengen von etwa 10 - etwa 1000, vorzugsweise 50-500 mg/kg Körpergewicht/24 h, gegebenenfalls in Form mehrerer Einzelgaben zur Erzielung der gewünschten Ergebnisse zu verabreichen. Eine Einzelgabe enthält den erfindungsgemässen Wirkstoff vorzugsweise in Mengen von etwa 15-etwa 150, insbesondere 10-100 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Der erfindungsgemässe Wirkstoff weist bei geringer Toxizität eine starke antimikrobielle Wirksamkeit auf. Diese Eigenschaften ermöglichen seine Verwendung als chemotherapeutischen Wirkstoff in der Medizin sowie als Stoff zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Der erfindungsgemässe Wirkstoff ist gegen ein sehr breites Spektrum von Mikroorganismen wirksam. Mit seiner Hilfe können gramnegative und grampositive Bakterien, bakterienähnliche Mikroorganismen bekämpft sowie die durch diese Erreger hervorgerufenen Erkrankungen verhindert, gebessert und/oder geheilt werden.

Besonders wirksam ist der erfindungsgemässe Wirkstoff gegen Bakterien und bakterienähnliche Mikroorganismen. Er ist daher besonders gut zur Prophylaxe und Chemotherapie von lokalen und systemischen Infektionen in der Human- und Tiermedizin geeignet, die durch diese Erreger hervorgerufen werden.

Beispielhaft können lokale und/oder systemische Erkrankungen behandelt und/oder verhindert werden, die durch die folgenden Erreger oder durch Mischungen der folgenden Erreger verursacht werden:

*Micrococaceae,* wie Staphylokokken, z.B. *Staphylococcus aureus, Staph. epidermidis, Staph. aerogenes* und *Gaffyka tetragena (Staph. = Staphylococcus)*;

*Lactobacteriaceae,* wie Streptokokken, z.B. *Streptococcus pyogenes,* α-bzw. β-hämolysierende Streptokokken, nicht (γ-)-hämolysierende Streptokokken, *Str. viridans, Str. faecalis* (Enterokokken), *Str. alalactiae, Str. lactis, Str. equi, Str. anaerobis* und *Diplococcus pneumoiae* (Pneumokokken) *(Str. = Streptococcus)*;

*Neisseriaceae,* wie Neisserien, z.B. *Neisseria gonorrhoeae,* (Gonokokken), *N. meningitidis* (Meningokokken), *N. catarrhalis* und *N. flava (N. = Neisseria);*
*Corynebacteriaceae,* wie Corynebakterien, z.B. *Corynebacterium diphtheriae, C. pyogenes, C. diphtheroides, C. acnes, C. parvum, C. bovis, C. renale, C. ovis, C. murisepticum, Listeria*-Bakterien, z.B. *Listeria monocytogenes, Erysipelothrix*-Bakterien, z.B. *Erysipelothrix insidiosa, Kurthia*-Bakterien, z.B. *Kurthia zopfii (C. = Corynebacterium);*
*Mycobacteriaceae,* wie Erreger von Mykobakteriosen, z.B. *Mycobacterium tuberculosis, M. bovis, M. avium,* sogenannte atypische Mykobakterien der Runyon-Gruppen I, II, III und IV, *M. leprae (M. = Mycobacterium);*
*Enterobacteriaceae,* wie *Escherichiae*-Bakterien der *Coli*-Gruppe: *Escherichia*-Bakterien, z.B. *Escherichia coli, Enterabacter*-Bakterien, z.B. *E. aerogenes, E. cloacae, Klebsiella*-Bakterien, z.B. *K. pneumoniae, K. ozaenae, Erwiniae,* z.B. *Erwinia sp. Serratia,* z.B. *Serratia marcescens (E. = Enterabacter), (K. = Klebsiella), Proteae*-Bakterien der *Proteus*-Gruppe: *Proteus,* z.B. *Proteus vulgaris, Pr. morganii, Pr. rettgeri, Pr. mirabilis, Providencia,* z.B. *Providencia sp. (Pr. = Proteus);*
*Salmonelleae: Salmonella*-Bakterien, z.B. *Salmonella paratyphi* A und B, *S. typhi, S. enteritidis, S. cholera suis, S. typhimurium (S. = Salmonella), Shigella*-Bakterien, z.B. *Shigella dysenteriae, Sh. ambigua, Sh. flexneri, Sh. boydii, Sh. sonnei (Sh. = Shigella);*
*Spirillaceae,* wie *Vibrio*-Bakterien, z.B. *Vibrio cholerae, V. proteus, V. fetus (V. = Vibrio), Spirillum*-Bakterien, z.B. *Spirillum minus;*
*Bacillaceae,* wie Aerobe Sporenbildner, z.B. *Bacillus anthracis, (B. subtilis, B. cereus) (B. = Bacillus),* Anaerobe Sporenbildner Clostridien, z.B. *Clostridium perfringens, Cl. septicum, Cl. oedematiens, Cl. histolyticum, Cl. tetani, Cl. botulinum (Cl. = Clostridium);*
Mykoplasmen, wie z.B. *Mycoplasma pneumoniae, M. hominis, M. suis pneumoniae, M. gallisepticum, M. hyorhinis (M. = Mycoplasma).*

Die obige Aufzählung von Erregern ist lediglich beispielhaft und keinesfalls beschränkend aufzufassen.

Als Krankheiten, die durch den erfindungsgemässen Wirkstoff verhindert, gebessert und/oder geheilt werden können, seien beispielsweise genannt:

Erkrankungen der Atmungswege und des Rachenraumes; Otitis, Pharyngitis, Pneumonie, Peritonitis, Pyelonephritis, Cystitis, Endocarditis, Systeminfektionen, Bronchitis, Arthritis, lokale Entzündungen, Hautinfektionen.

Der erfindungsgemässe Wirkstoff kann auch in allen Bereichen der Tierzucht und Tierhaltung als Mittel zur Förderung und Beschleunigung des Wachstums und zur Verbesserung der Futterverwertung bei gesunden und kranken Tieren verwendet werden.

Die Wirksamkeit des erfindungsgemässen Wirkstoffes ist hierbei weitgehend unabhängig von der Art und dem Geschlecht der Tiere. Besonders wertvoll erweist sich der erfindungsgemässe Wirkstoff bei der Aufzucht und Haltung von Jung- und Masttieren. Als Tiere, bei denen der erfindungsgemässe Wirkstoff zur Förderung und Beschleunigung des Wachstums und zur Verbesserung der Futterverwertung eingesetzt werden kann, seien beispielsweise folgende Nutz- und Ziertiere genannt:

Warmblüter wie Rinder, Schweine, Pferde, Schafe, Ziegen, Katzen, Hunde, Kaninchen, Pelztiere, z.B. Nerze und Chinchilla, Geflügel, z.B. Hühner, Gänse, Enten, Truthähne, Tauben, Papageien und Kanarienvögel und Kaltblüter, wie Fische, z.B. Karpfen und Reptilien, z.B. Schlangen.

Die Menge des erfindungsgemässen Wirkstoffes, die den Tieren zur Erreichung des gewünschten Effektes verabreicht wird, kann wegen der günstigen Eigenschaften des Wirkstoffes weitgehend variiert werden. Sie liegt vorzugsweise bei etwa 5-500, insbesondere 10 bis 100 mg/kg Körpergewicht/d. Die Dauer der Verabreichung kann von wenigen Stunden oder Tagen bis zu mehreren Jahren betragen. Die passende Menge Wirkstoff sowie die passende Dauer der Verabreichung hängen insbesondere von der Art, dem Alter, dem Geschlecht, dem Gesundheitszustand und der Art der Haltung der Tiere ab und sind durch jeden Fachmann leicht zu ermitteln.

Der erfindungsgemässe Wirkstoff wird den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmässigen oder unregelmässigen Abständen oral oder parenteral erfolgen. Aus Zweckmässigkeitsgründen ist in den meisten Fällen eine orale Verabreichung, insbesondere im Rhythmus der Nahrungs- und/oder Getränkeaufnahme der Tiere, vorzuziehen.

Der erfindungsgemässe Wirkstoff kann als reiner Stoff oder in formulierter Form, also in Mischung mit nichttoxischen inerten Trägerstoffen beliebiger Art, z.B. mit Trägerstoffen und in Formulierungen, wie sie weiter oben bei den pharmazeutischen Zubereitungen beschrieben sind, verabreicht werden.

Der erfindungsgemässe Wirkstoff kann gegebenenfalls in formulierter Form auch zusammen mit pharmazeutischen Wirkstoffen, Mineralsalzen, Spurenelementen, Vitaminen, Eiweissstoffen, Fetten, Farbstoffen und/oder Geschmacksstoffen in geeigneter Form verabreicht werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwassers zugegeben werden.

Der erfindungsgemässe Wirkstoff kann nach üblichen Methoden durch einfaches Mischen als reiner Stoff, vorzugsweise in fein verteilter Form oder in formulierter Form in Mischung mit essbaren nichttoxischen Trägerstoffen, gegebenenfalls auch in Form eines Praemix oder eines Futterkonzentrates, dem Futter und/oder Trinkwasser beigefügt werden.

Das Futter und/oder Trinkwasser kann beispielsweise den erfindungsgemässen Wirkstoff in einer Konzentration von etwa 5-500, insbesondere 10 bis 100 ppm (Gewicht) enthalten. Die optimale Höhe der Konzentration des Wirkstoffs in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Aufbaustoffen einschliesslich Vitaminen und Mineralstoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten den erfindungsgemässen Wirkstoff neben essbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten kann der Wirkstoff gegebenenfalls auch durch seine Oberfläche bedeckenden geeigneten Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das den erfindungsgemässen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin/Mineral-Mischung und 2,5 g Wirkstoff-Praemix ergeben nach sorgfältigen Mischen 1 kg Futter.

Die Vitamin/Mineral-Mischung besteht aus: 6000 IE Vitamin A, 1000 IE Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7H_2O$, 100 mg $FeSO_4 \times 7H_2O$ und 20 mg $CuSO_4 \times 5H_2O$.

Der Wirkstoff-Praemix enthält den erfindungsgemässen Wirkstoff in der gewünschten Menge, z.B. 100 mg und zusätzlich, 1 g DL-Methionin sowie so viel Sojabohnenmehl, dass 2,5 g Praemix entstehen.

Beispiel für die Zusammensetzung eines Schweineaufzuchtfutters, das den erfindungsgemässen Wirkstoff enthält:

630 g Futtergetreideschrot (zusammengesetzt aus 200 g Mais, 150 g Gerste-, 150 g Hafter- und 130 g Weizenschrot), 80 g Fischmehl, 60 g Sojaschrot, 60 g Tapiokamehl, 38 g Bierhefe, 50 g Vitamin/Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die gute antimikrobielle Wirksamkeit des erfindungsgemässen Antibiotikums kann durch die folgenden Versuche demonstriert werden:

a) *In vitro*-Versuche:

Das erfindungsgemässe Antibiotikum wurde mit Müller-Hinton-Nährbrühe unter Zusatz von 1% Glucose auf einen Gehalt von 200 µg/ml verdünnt. In der Nährlösung befanden sich jeweils $1 \times 10^5$ bis $2 \times 10^5$ Bakterien/ml. Die Röhrchen mit diesem Ansatz wurden jeweils 18 h bebrütet und anschliessend wurde der Trübungsgrad bestimmt. Trübungsfreiheit gibt Wirkung an. Bei der Dosierung von 200 µg/ml waren die folgenden Bakterienkulturen trübungsfrei:

*Escherichia coli* 14; *Escherichia coli* C 165; *Proteus vulgaris* 1017; *Klebsiella* K 10; *Klebsiella* 63; *Salmonella sp.*; *Shigella sp.*; *Enterobacter sp.*; *Serratia sp.*; *Proteus* indolnegativ *sp.*; *Proteus* indolpositiv *sp.*; *Pasteurella pseudotuberculsis*; *Staphylococcus aureus* 133; *Neisseria catarrhalis sp.*; *Diplococcus pneumoniae sp.*; *Streptococcus pyogenes* W.; *Enterococcus sp.*; *Lactobacillus sp.*; *Corynebacterium diphtheriae gravis*; *Corynebacterium pyogenes* M; *Clostridium botulinum*; *Clostridium tetani*; *Pseudomonas aeruginosa sp.*; *Aeromonas hydrophila sp.*; *Mycoplasma sp.* (*Sp.* = *species* = nicht näher identifizierte, jedoch charakteristische Stämme).

b) *In vivo*-Versuche:

Aus der folgenden Tabelle 1 geht die Wirkung des erfindungsgemässen Antibiotikums gegen eine Reihe von Bakterien im Tierversuch mit der weissen Maus hervor. Die weissen Mäuse vom Stamm $CF_1$ wurden intraperitoneal mit der jeweils angegebenen Bakterienart infiziert.

Tabelle 1

Tierversuch mit der weissen Maus.
Bestimmung der $ED_{50}$ nach 24 h.

| Keim | Dosis in mg des erfindungsgemässen Antibiotikums/kg Körpergewicht |
|---|---|
| *Escherichia coli* 165 | 2 × 600 |
| *Staphylococcus aureus* | 1 × 400 |

Therapie: Einmalig 30 min nach Infektion, subcutan.
Zweimalig 30 min nach 90 min nach Infektion.

Die $ED_{50}$ ist die Dosis, bei der 50% der infizierten Tiere nach 24 h noch überleben.

Die gute Wirksamkeit des erfindungsgemässen Antibiotikums als Mittel zur Förderung des Wachstums bei Tieren kann durch folgenden Versuch demonstriert werden:

Bei Fütterungsversuchen wurde die Substanz dem Futter untergemischt und an Küken verfüttert (Futter *ad libitum*). Die Dosis betrug 10 bzw. 20 ppm. Verglichen wurde gegen eine Negativkontrolle (Futter ohne Zusätze). Versuchsdauer: 14d.

*Versuch*

| Gruppe | Gewichtszuwachs (%) | Futterverbrauch (%) | Futterverwertung (%) |
|---|---|---|---|
| Kontrolle | 100 | 100 | 100 |
| Erfindungsgemässes Antibiotikum | | | |
| 10 ppm | 102,8 | 102,8 | 100 |
| 20 ppm | 103,5 | 102,3 | 98,5 |

Die Herstellung des erfindungsgemässen Antibiotikums kann durch die folgenden Beispiele erläutert werden.

*Beispiel 1*

Auf Schrägagar der Zusammensetzung (Gewichtsprozente):

| | |
|---|---|
| Pepton | 0,25 |
| Caseinhydrolisat, sauer | 0,25 |
| $K_2HPO_4 \cdot 3H_2O$, p.a. | 0,1 |
| KCL, p.a. | 0,05 |
| $MgSO_4$, p.a. | 0,05 |
| $FeSO_4$, p.a. | 0,01 |
| Rohrzucker | 3,0 |
| Agar | 2,0 |
| Wasser | ad 100 |

gewachsenes Mycel des Actinoplanes-Stammes SE-73/34d (Kl.) wird zur Vorkultur auf je 140 ml sterile, in 1-l-Erlenmeyer-Kolben befindliche Nährlösung (im folgenden Nährlösung A genannt) der Zusammensetzung (Gewichtsprozente):

| | |
|---|---|
| Sojamehl, entfettet | 3,0 |
| Glycerin, rein | 3,0 |
| $CaCO_3$, p.a. | 0,2 |
| Wasser | ad 100 |

und Hydroxylgruppen enthaltendes neutrales Polyol (1 Tropfen auf 140 ml Nährlösung zur Entschäumung) überimpft.

Nach viertägiger Kultivierung des Stammes auf der Rundschüttelmaschine bei 28°C wird jeweils 1 ml der so hergestellten Schüttelkultur auf je 140 ml steriler, in 1-l-Erlenmeyer-Kolben befindliche Nährlösung (im folgenden Nährlösung 15 genannt) der Zusammensetzung (Gewichtsprozente):

| | |
|---|---|
| Sojamehl entfettet | 0,0 |
| Glycerin, rein | 2,0 |
| Glucose, rein | 1,0 |
| $CaCO_3$, p.a. | 0,2 |
| Wasser | ad 100 |

und Hydroxylgruppen enthaltendes neutrales Polyol (1 Tropfen auf 140 ml Nährlösung zur Entschäumung) überimpft.

Nach 5tägiger Kultivierung des Stammes auf der Rundschüttelmaschine bei 28°C werden Proben der Kulturen zentrifugiert. Die klare, überstehende Lösung wird im Agar-Plattenlochtest gegen *Bacillus subtilis* ATCC 6633 getestet. Das in der Kulturlösung befindliche erfindungsgemässe Antibiotikum verursacht Wachstumshemmzonen von etwa 18 mm Durchmesser. Die Isolierung des erfindungsgemässen Antibiotikums aus den Kulturbrühen geschieht wie im Beispiel 3 beschrieben.

*Beispiel 2*

Je 8 l Nährlösung der Zusammensetzung (Gewichtsprozente):

| | |
|---|---|
| Sojamehl, entfettet | 2,0 |
| Glycerin | 2,0 |
| Dextrin | 1,0 |
| $CaCO_3$ | 0,2 |
| Wasser | ad 100 |

und 2 ml Entschäumer werden in Glasfermenter mit Rührwek und Belüftungseinrichtung abgefüllt, mit Natronlauge auf pH 7,0 gestellt und bei 120°C sterilisiert. Nach Abkühlen der Lösung werden die Fermenter mit je 140 ml von 4d in Nährlösung A gewachsenen Schüttelkulturen des Actinoplanaceenstammes SE-73/34d (Kl.) geimpft, mit etwa 2 l Luft/min bei etwa 150 Umdrehungen des Rührwerks/min belüftet und bei einer Temperatur von 28°C gehalten. 18 h nach Beginn der Kultivierung werden die Umdrehungen des Rührwerks auf 3000 tr/min erhöht. Nach 90 h wird die Kultivierung beendet.

Eine Probe der Kulturbrühe wird mit dem gleichen Volumen Aceton versetzt, stark umgeschüttelt und nach 30 min zentrifugiert. In der überstehenden Lösung wird das in der Kultur gebildete Antibiotikum in folgender Weise hochdruckflüssigkeits-chromatographisch bestimmt:

Gerät: Knauer
Säule: Stahlsäule, Länge 25 cm, I.D. 4 mm
Mobile Phase: Methanol/Wasser 80:20 (V/V)
Stationäre Phase: $C_{18}$, 7,5 $\mu$
Druck: $1,8 \cdot 10^7$ Pa
Fluss: 1,5 ml/min
Detektion: $\lambda$ = 365 nm
Aufgabemenge: 125 $\mu$l einer 0,1 bzw. 0,01%igen Lösung

Aufgabesystem: Probenschleife

Die Kulturbrühe enthielt 228 mg/l Wirkstoff.

Analog vorstehender analytischer Bestimmung wurde die präparative Reindarstellung des erfindungsgemässen Antibiotikums mit Hilfe der Hochdruckflüssigkeits-chromatographie durchgeführt, wobei folgende Bedingungen geändert wurden:

Druck: $7{,}5 \cdot 10^6$ Pa
Fluss: 4 ml/min
Detektion: $\lambda = 308$ nm, $\lambda = 365$ nm
Aufgabemenge: 10 mg/50 µl-System

Aus 300 mg Einsatzmenge wurden 102 mg reines Antibiotikum gewonnen.

*Beispiel 3*

Die gemäss Beispiel 2 erhaltenen Kulturbrühen von 5 Glasfermentern (5 × 8 l) werden vereinigt, mit 48 l Aceton versetzt, bei ca. 25°C 1 h gerührt und anschliessend zentrifugiert. Der klare, vom extrahierten Mycel befreite Überstand wird bei 20-35°C im Vakuum auf etwa 35 l eingeengt, mit Natronlauge auf pH 6,5 eingestellt und mit 18 l Äthylacetat extrahiert.

Die organische Phase wird mit 500 g Natriumsulfat getrocknet, im Vakuum auf ca. 1 l eingedampft und mit 2 l Cyclohexan versetzt.

Durch Abfiltrieren des ausgefallenen Niederschlages erhält man nach Trocknen an der Luft 8 g des rohen erfindungsgemässen Antibiotikums.

*Beispiel 4*

Reindarstellung des erfindungsgemässen Antibiotikums durch präparative Dünnschichtchromatographie (DC).

Im Dünnschichtchromatogramm des nach Beispiel 3 erhaltenen rohen Antibiotikums befindet sich das erfindungsgemässe Antibiotikum in der Hauptzone mit dem $R_F$-Wert 0,34. (Fliessmittel Nr. 5 aus Tabelle 2, Kieselgelplatten, Fa. Merck, Darmstadt, BRD, Kieselgel 60 $F_{254}$.) 1,00 g des rohen Antibiotikums werden auf 20 Kieselgelplatten (20 × 20 cm) (Kieselgel PSC $F_{254}$, Fa. Merck) getrennt (Fliessmittel Nr. 5 aus Tabelle 2).

Isoliert wird die Hauptzone mit dem $R_F$-Wert 0,34.

Ausbeute: 327 mg.

*Beispiel 5*

Reindarstellung des erfindungsgemässen Antibiotikums durch Säulenchromatographie.

3,5 g des rohen gemäss Beispiel 3 erhaltenen Antibiotikums werden an einer 100 cm langen Säule mit 5 cm Durchmesser gefüllt mit neutralem Kieselgel (Kieselgel 60, Korngrösse unter 0,063, Fa. Merck, Darmstadt), in Fliessmitel Nr. 5 aus Tabelle 2 chromatographiert.

Aufgefangen wird die bei 365 nm absorbierende Hauptfraktion. Das Eluat wird im Vakuum eingedampft und der Rückstand aus Essigsäureäthylester/Petroläther umgefällt. Das Antibiotikum wird bei 100°C 4d im Hochvakuum bei 1,33 Pa getrocknet.

Ausbeute: 1,8 g.

*Beispiel 6*

Reindarstellung durch Craig-Verteilung.

50 g eines nach dem in Beispiel 3 beschriebenen Verfahren hergestellten Rohproduktes werden einer Craig-Verteilung unterworfen, wobei eine Apparatur der Fa. Labortec F. Schmidiger, Basel (Schweiz) mit 250 cm³ Phasenvolumen und ein Lösungsmittelgemisch aus Petroläther, Essigester, Dimethylformamid und Wasser im Verhältnis 1,5:8,5:5:5 (Volumen) verwendet wird.

Es werden 180 Verteilungsschritte durchgeführt, wobei nach 60 Schritten die Oberphase fraktioniert entnommen wird. Das Antibiotikum befindet sich in den Fraktionen 21-68. Die aufgefangene Oberphase wird mit Wasser gewaschen, im Vakuum weitgehend eingedampft und mit 100 ml Essigsäureäthylester versetzt. Durch Zugabe von 1 l Diäthyläther wird das Antibiotikum ausgefällt. Es wird abfiltriert und im Hochvakuum bei 100°C und 1,33 Pa 4d getrocknet.

Ausbeute: 17,0 g.

Der in den Beispielen aufgeführte Plattendiffusionstest wird wie folgt vorgenommen (vgl. P. Klein, „Bakteriologische Grundlagen der Chemotherapeutischen Laboratoriumspraxis", Springer Verlag, Göttingen, 1957, S. 86 ff.):

In eine durch *Bacillus subtilis* ATCC 6633 infizierte Agar-Platte werden Löcher gestanzt. Das zu prüfende Material wird als wässrige Lösung in diese Löcher gegeben. Anschliessend wird bei 37°C ca. 16 h betrütet. Hemmhöfe zeigen Wirksamkeit gegen den verwendeten Keim an. Aus der Grösse der Hemmhöfe kann auf den Gehalt an dem erfindungsgemässen Antibiotikum geschlossen werden.

**Patentansprüche**

1. Antibiotikum der Formel:

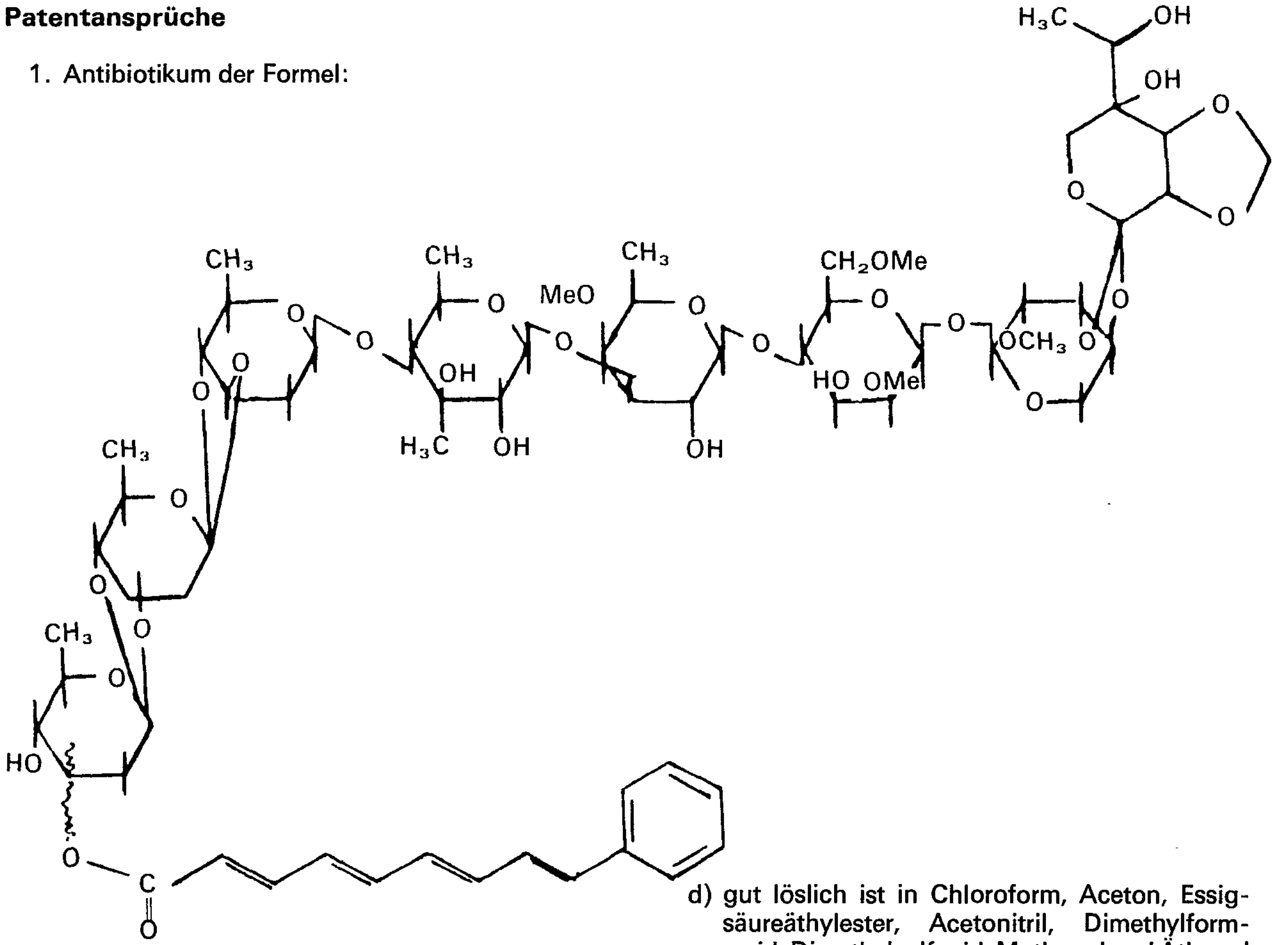

2. Antibiotikum, welches
a) ein Neutralstoff ist und bei der Elektrophorese nicht wandert;
b) aus Kohlenstoff, Wasserstoff und Sauerstoff besteht;
c) im UV-Spektrum ein Maximum bei λ = 365 nm zeigt;
d) gut löslich ist in Chloroform, Aceton, Essigsäureäthylester, Acetonitril, Dimethylformamid, Dimethylsulfoxid, Methanol und Äthanol und wenig löslich in Wasser, Diäthyläther, Petroläther und Cyclohexan ist;
e) Methoxygruppen enthält;
f) bei der Hydrolyse mit 10 Gew.% Schwefelsäure bei 80°C reduzierende Zucker abspaltet;
g) einen Schmelzpunkt von 164-172°C unter Zersetzung hat;
h) mit einer elementaranalytisch bestimmten Zusammensetzung aus etwa 55,6% C, 6,9% H und 37,8% O,
i) einem IR-Spektrum gemäss Fig. 2,
j) einem $^1$H-NMR-Spektrum gemäss Fig. 3, und
k) einem $^{13}$C-NMR-Spektrum gemäss Fig. 4.

3. Verfahren zur Herstellung eines Antibiotikums, welches
a) ein Neutralstoff ist und bei der Elektrophorese nicht wandert;
b) aus Kohlenstoff, Wasserstoff und Sauerstoff besteht;
c) im UV-Spektrum ein Maximum bei λ = 365 nm zeigt;
d) gut löslich ist in Chloroform, Aceton, Essigsäureäthylester, Acetonitril, Dimethylformamid, Dimethylsulfoxid, Methanol und Äthanol und wenig löslich in Wasser, Diäthyläther, Petroläther und Cyclohexan ist;
e) Methoxygruppen enthält;
f) bei der Hydrolyse mit 10 Gew.% Schwefelsäure bei 80°C reduzierende Zucker abspaltet;
g) einen Schmelzpunkt von 164-172°C unter Zersetzung hat;
h) mit einer elementaranalytisch bestimmten Zusammensetzung aus etwa 55,6% C, 6,9% H und 37,8% O, und
i) einem IR-Spektrum gemäss Fig. 2,
j) einem $^1$H-NMR-Spektrum gemäss Fig. 3,
k) einem $^{13}$C-NMR-Spektrum gemäss Fig. 4,

dadurch gekennzeichnet, dass man den Actinoplanesstamm SE-73/34 d (Kl.) unter aeroben Bedingungen in einem üblichen Kohlenstoff-, Stickstoff- und Mineralstoffe enthaltenden Nährmedium züchtet und das Antibiotikum nach üblichen Methoden aus der Kulturbrühe und/oder dem Mycelium isoliert und gegebenenfalls reinigt.

4. Verfahren gemäss Anspruch 3, bei welchem die Züchtungstemperatur zwischen etwa 20 und etwa 40°C liegt.

5. Verfahren gemäss Anspruch 3, bei welchem der pH-Wert der wachsenden Kultur bei etwa pH 6 bis etwa pH 8 liegt.

6. Arzneimittel, gekennzeichnet durch einen Gehalt an dem Antibiotikum gemäss den Ansprüchen 1 und 2.

7. Verwendung des Antibiotikums gemäss den Ansprüchen 1 und 2 zur Förderung des Wachstums und zur Verbesserung der Futterverwertung bei Tieren.

8. Tierfutter, enthaltend das Antibiotikum gemäss den Ansprüchen 1 und 2.

9. Actinoplanesstamm SE-73/34 d (Kl.) (ATCC 31440).

---

## Revendications

1. L'antibiotique de formule:

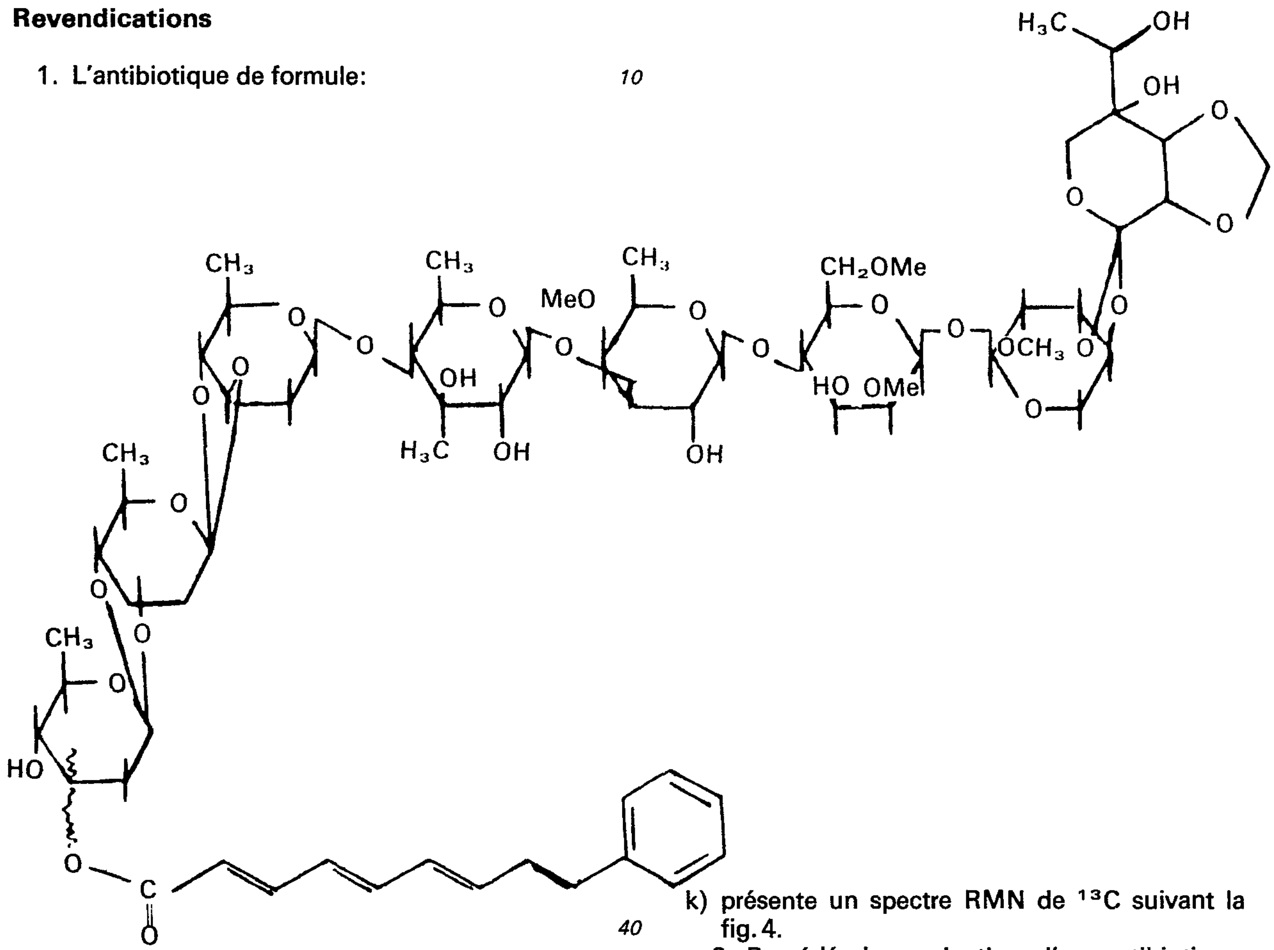

2. Antibiotique, qui

a) est une substance neutre et n'émigre pas dans l'électrophorèse;
b) est formé de carbone, d'hydrogène et d'oxygène;
c) présente un maximum à $\lambda$ = 365 nm dans le spectre ultraviolet;
d) se dissout bien dans le chloroforme, l'acétone, l'ester éthylique de l'acide acétique, l'acétonitrile, le diméthylformamide, le diméthylsulfoxyde, le méthanol et l'éthanol, et se dissout peu dans l'eau, l'éther de diéthyle, l'éther de pétrole et le cyclohexane;
e) porte des groupes méthoxy;
f) libère des sucres réducteurs par hydrolyse avec 10% en poids d'acide sulfurique à 80°C;
g) a un point de fusion de 164-172°C avec décomposition;
h) a une composition, déterminée par l'analyse élémentaire, d'environ 55,6% de C, 6,9% de H et 37,8% de O,
i) présente un spectre IR suivant la fig. 2,
j) présente un spectre RMN de $^{1}H$ suivant la fig. 3, et
k) présente un spectre RMN de $^{13}C$ suivant la fig. 4.

3. Procédé de production d'un antibiotique, qui,

a) est une substance neutre et n'émigre pas dans l'électrophorèse;
b) est formé de carbone, d'hydrogène et d'oxygène;
c) présente un maximum à $\lambda$ = 365 nm dans le spectre ultraviolet;
d) se dissout bien dans le chloroforme, l'acétone, l'ester éthylique de l'acide acétique, l'acétonitrile, le diméthylformamide, le diméthylsulfoxyde, le méthanol et l'éthanol, et se dissout peu dans l'eau, l'éther de diéthyle, l'éther de pétrole et le cyclohexane;
e) porte des groupes méthoxy;
f) libère des sucres réducteurs par hydrolyse avec 10% en poids d'acide sulfurique à 80°C;
g) a un point de fusion de 164-172°C avec décomposition;
h) a une composition, déterminée par l'analyse élémentaire, d'environ 55,6% de C, 6,9% de H et 37,8% de O,
i) présente un spectre IR suivant la fig. 2,
j) présente un spectre RMN de $^{1}H$ suivant la fig. 3, et

k) présente un spectre RMN de $^{13}C$ suivant la fig. 4,

caractérisé en ce qu'on cultive la souche d'actinoplanes SE-73/34 d (Kl.) dans des conditions aérobies dans un milieu nutritif usuel contenant du carbone, de l'azote et des substances minérales, et en ce que l'antibiotique est, par des opérations classiques, isolé du bouillon de culture et/ou du mycélium et éventuellement purifié.

4. Procédé suivant la revendication 3, dans lequel la température de culture se situe entre environ 20 et environ 40°C.

5. Procédé suivant la revendication 3, dans lequel la valeur du pH de la culture en cours de croissance se situe de pH 6 environ à pH 8 environ.

6. Médicament, caractérisé par une teneur en l'antibiotique suivant les revendications 1 et 2.

7. Utilisation de l'antibiotique suivant les revendications 1 et 2 pour activer la croissance et améliorer l'assimilation des aliments chez les animaux.

8. Aliments pour animaux, contenant l'antibiotique suivant les revendications 1 et 2.

9. Souche d'actinoplanes SE-73/34 d (Kl.) (ATCC 31440).

---

**Claims**

1. An antibiotic of the formula:

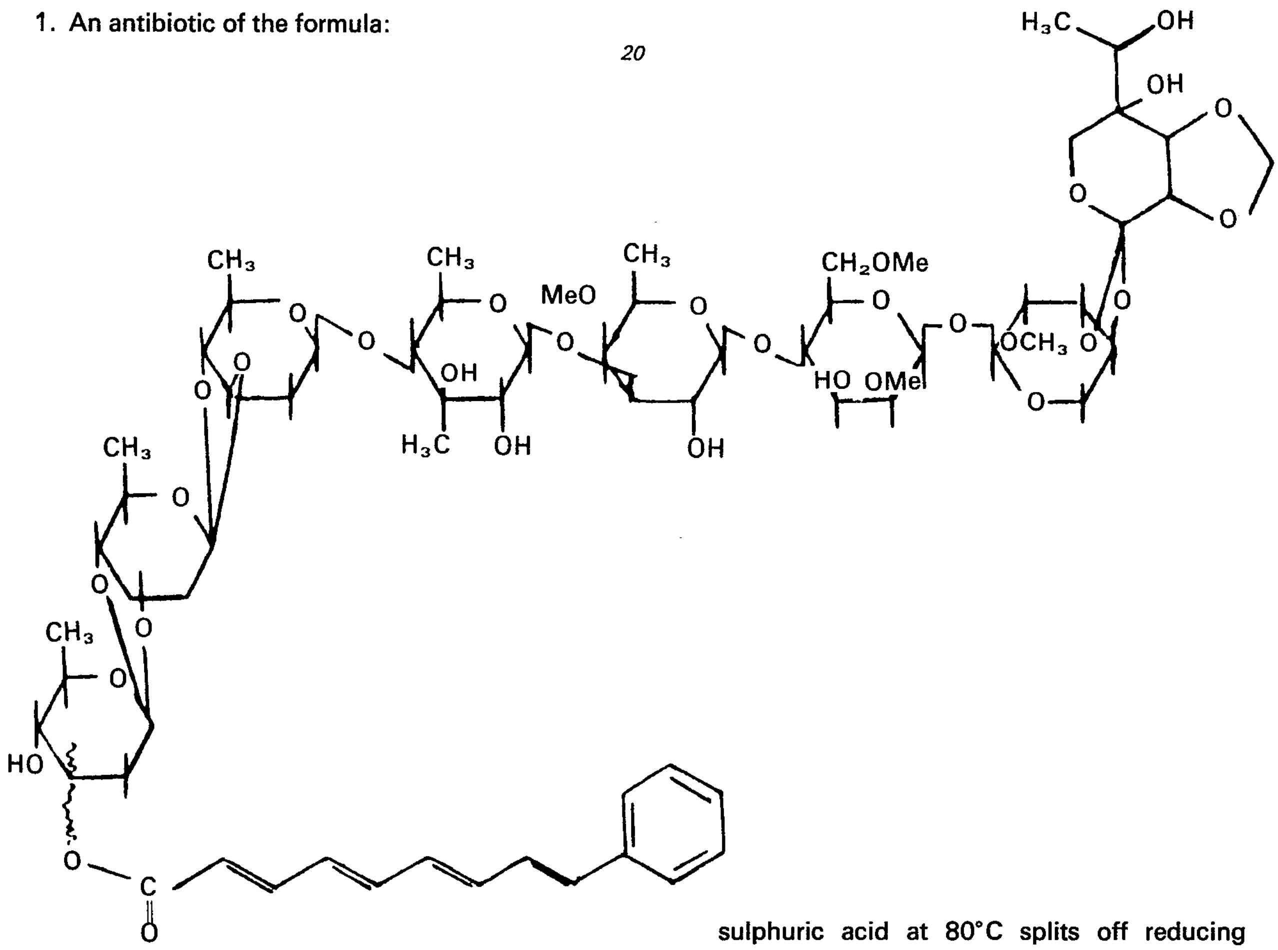

2. An antibiotic which
a) is a neutral compound and does not migrate on electrophoresis;
b) consists of carbon, hydrogen and oxygen;
c) shows a maximum, in the UV spectrum, at $\lambda$ = 365 nm;
d) is readily soluble in chloroform, acetone, ethyl acetate, acetonitrile, dimethylformamide, dimethyl sulphoxide, methanol and ethanol, and sparingly soluble in water, diethyl ether, petroleum ether and cyclohexane;
e) contains methoxy groups;
f) on hydrolysis with 10% strength by weight sulphuric acid at 80°C splits off reducing sugars;
g) has a melting point of 164-172°C with decomposition;
h) has a composition of about 55.6% C, 6.9% H and 37.8% O, as determined by elementary analysis,
i) an IR spectrum according to Fig. 2,
j) a $^{1}H$-NMR spectrum according to Fig. 3, and
k) a $^{13}C$-NMR spectrum according to Fig. 4.

3. Process for the preparation of an antibiotic which
a) is a neutral compound and does not migrate on electrophoresis;
b) consists of carbon, hydrogen and oxygen;

c) shows a maximum, in the UV spectrum, at λ = 365 nm;
d) is readily soluble in chloroform, acetone, ethyl acetate, acetonitrile, dimethylformamide, dimethyl sulphoxide, methanol and ethanol, and sparingly soluble in water, diethyl ether, petroleum ether and cyclohexane;
e) contains methoxy groups;
f) on hydrolysis with 10% strength by weight sulphuric acid at 80°C splits off reducing sugars;
g) has a melting point of 164-172°C with decomposition;
h) has a composition of about 55.6% C, 6.9% H and 37.8% O, as determined by elementary analysis,
i) an IR spectrum according to Fig. 2,
j) a $^1H$-NMR spectrum according to Fig. 3, and
k) a $^{13}C$-NMR spectrum according to Fig. 4,

characterised in that the Actinoplanes strain SE-73/34 d (Kl.) is cultured under aerobic conditions in a customary nutrient medium containing carbon, nitrogen and mineral substances, and characterised in that the antibiotic is isolated from the culture broth and/or the mycelium in accordance with customary methods and is purified if necessary.

4. Process according to claim 3, in which the culture temperature is between about 20 to about 40°C.

5. Process according to claim 3, in which the pH value of the growing culture is about pH 5 to about pH 8.

6. Medicaments, characterised in that they contain the antibiotic according to claims 1 and 2.

7. Use of the antibiotic according to claims 1 and 2 for promoting the growth and improving the feed utilisation of animals.

8. Animal feeds containing the antibiotic according to claims 1 and 2.

9. The Actinoplanes strain SE-73/34 d (Kl.) (ATCC 31440).

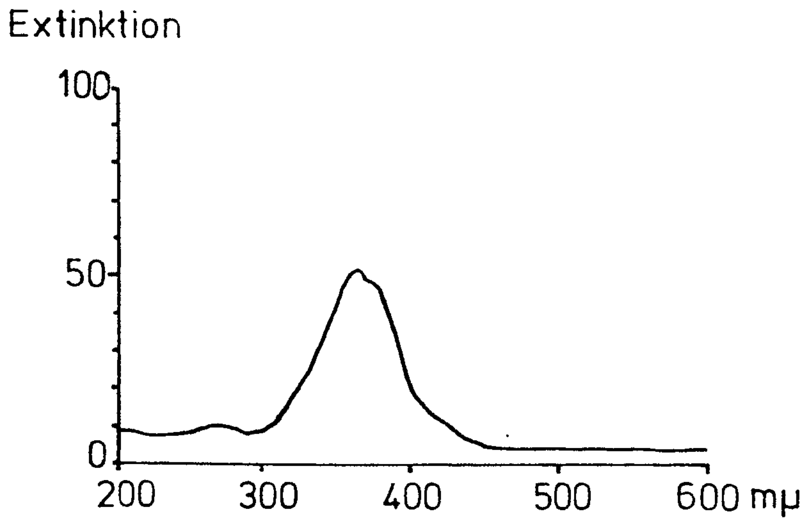
Extinktion
100
50
0
200
300
400
500
600 mμ

FIG. 1

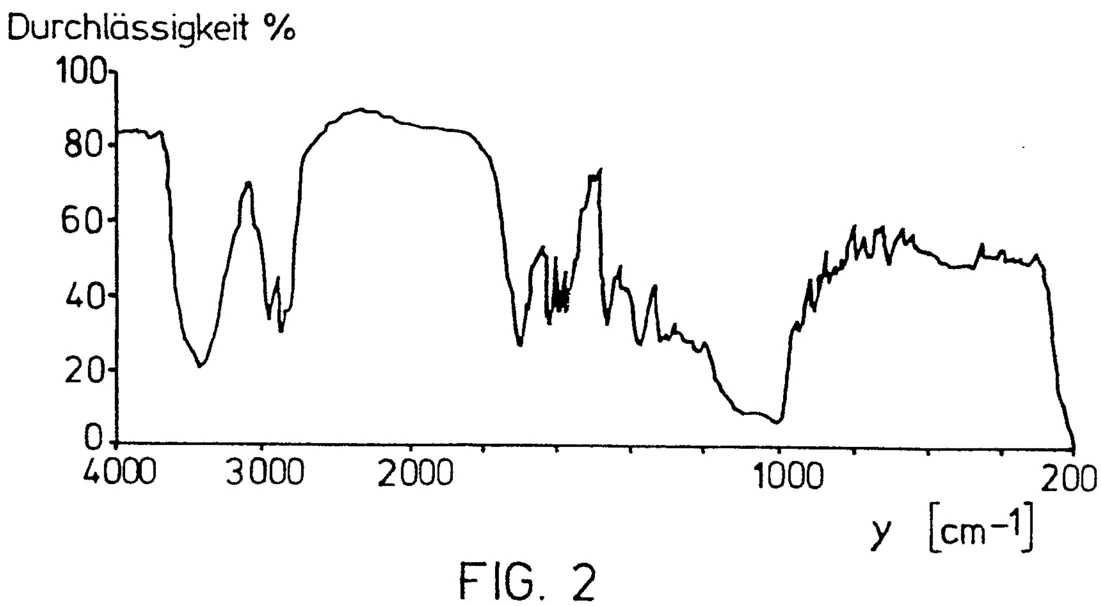
Durchlässigkeit %
100
80
60
40
20
0
4000
3000
2000
1000
200
y [cm-1]

FIG. 2

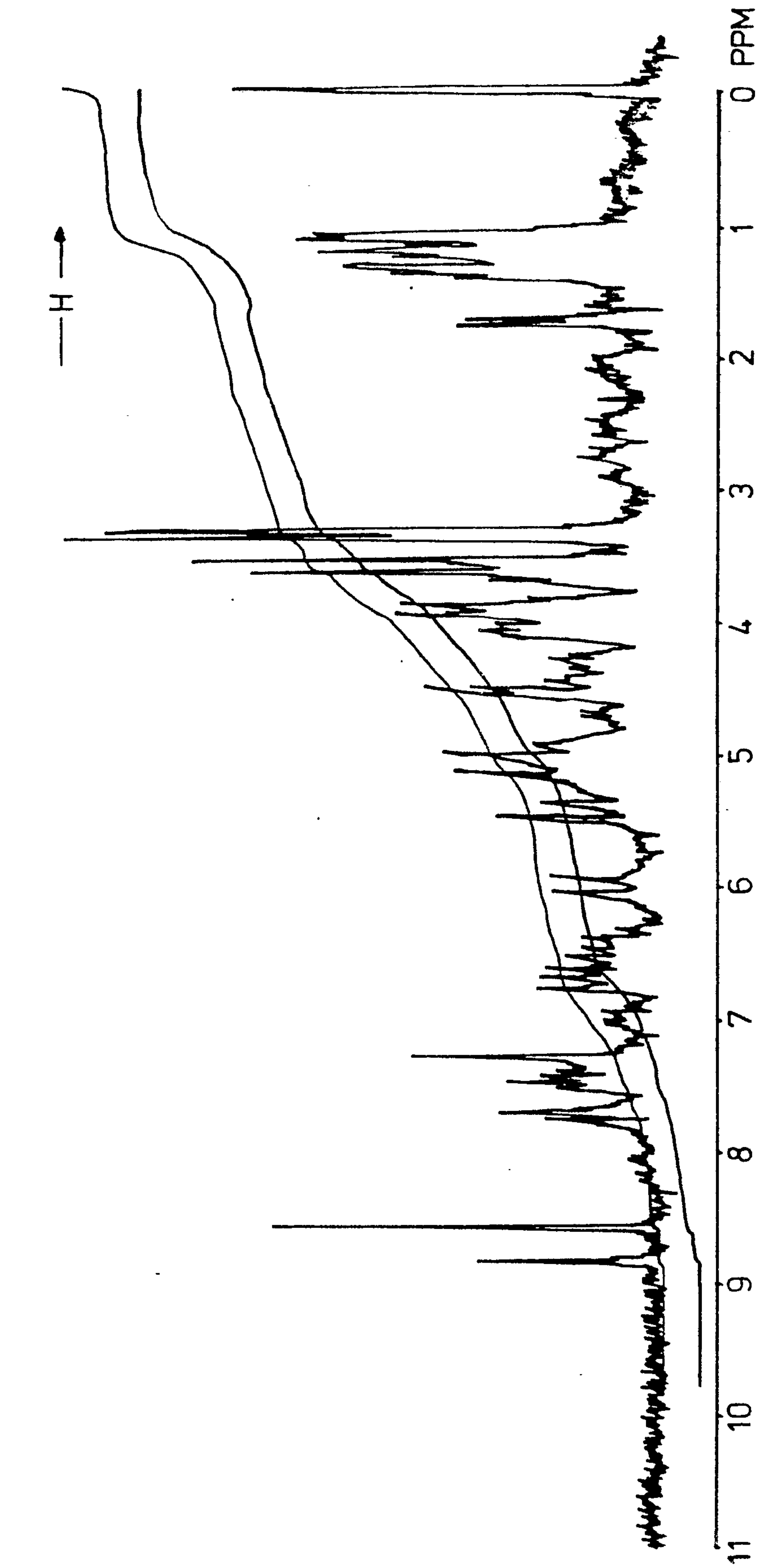


FIG. 3

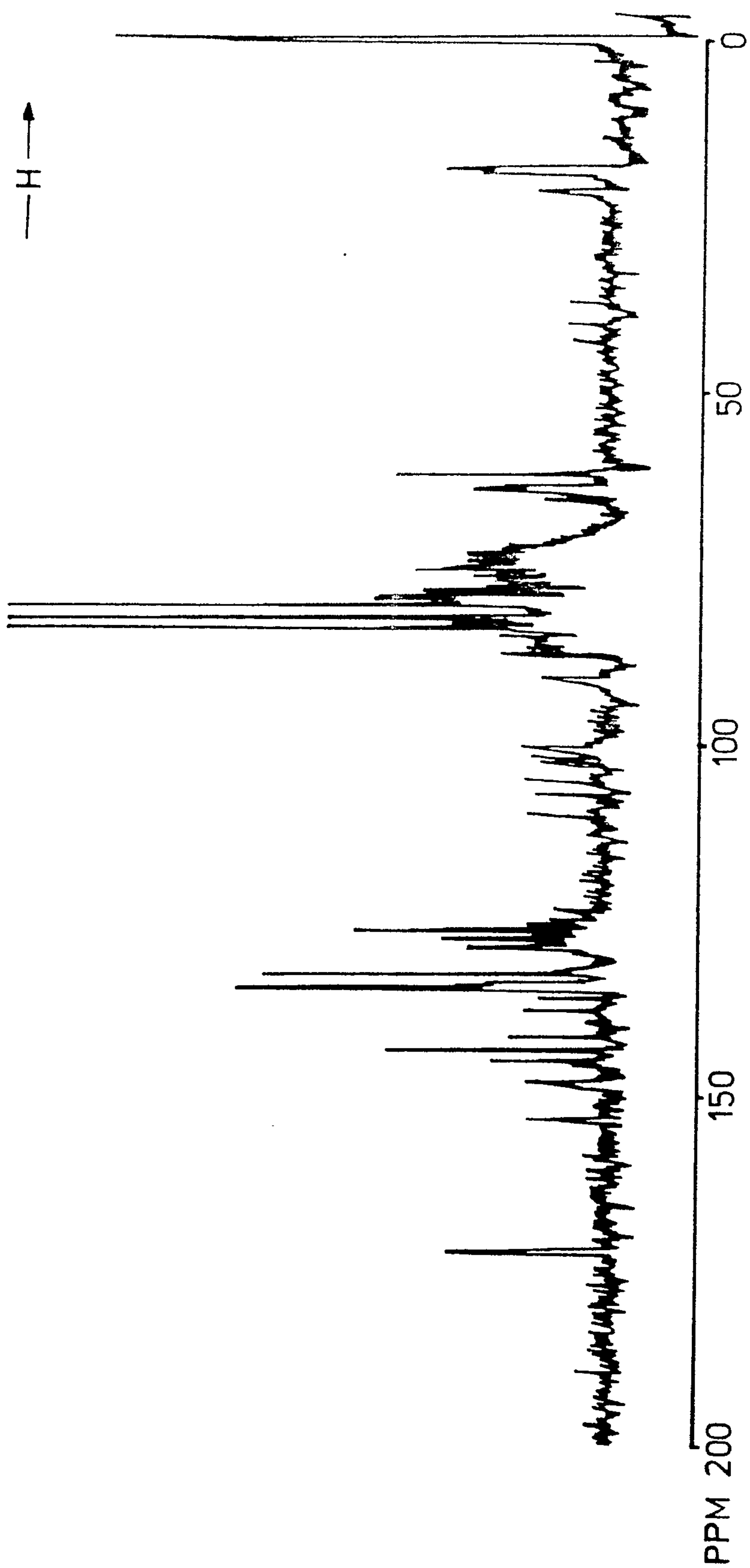


FIG. 4